# FASCICULE DE BREVET EUROPEEN

(11) **EP 3 139 790 B1**
(45) Date de publication et mention de la délivrance du brevet: **25.04.2018**
(21) Numéro de dépôt: 15729536.1
(22) Date de dépôt: 05.05.2015
(51) Int. Cl.: A45D 34/00

(54) **ENSEMBLE DE DISTRIBUTION ET D'APPLICATION DE PRODUIT FLUIDE**
ANORDNUNG ZUR AUSGABE UND APPLIKATION EINES FLUIDPRODUKTS
ASSEMBLY FOR DISPENSING AND APPLYING A FLUID PRODUCT

(30) Priorité: 07.05.2014 FR 1454114
(43) Date de publication de la demande: 15.03.2017
(73) Titulaire: APTAR France SAS, 27110 Le Neubourg (FR)
(72) Inventeur: DUQUET, Frédéric, F-78121 Crespières (FR); PIERRE, Christophe, F-27180 Saint Sebastien de Morsent (FR)
(74) Mandataire: CAPRI
(86) Numéro de dépôt international: PCT/FR2015/051185
(87) Numéro de publication internationale: WO 2015/170048

(56) Documents cités:
- WO-A1-2013/121145
- GB-A- 2 449 141
- US-A1- 2011 190 672

## Description

La présente invention concerne un ensemble de distribution et d'application de produit fluide comprenant un corps de réception creux, une surface d'application de produit fluide montée à une extrémité supérieure du corps de réception et destinée à venir en contact avec une cible, telle que la peau, et un distributeur de produit fluide comprenant un réservoir, une pompe et un embout de distribution définissant une surface de sortie dans laquelle débouche un orifice de distribution de produit fluide. L'ensemble comprend avantageusement un module de génération d'ondes qui émet des ondes et les transmet à la surface d'application. Ainsi l'ensemble permet d'appliquer un produit fluide sur cible tel que la peau, et simultanément, postérieurement ou antérieurement soumettre la peau à des ondes au travers de la surface d'application. Le domaine d'application privilégié de la présente invention est bien entendu celui de la cosmétique, mais peut aussi être celui de la pharmacie.

Dans l'art antérieur on connait déjà le document WO2013/121145 qui décrit un ensemble de distribution et d'application de ce type. Il comprend une tête d'application définissant la surface d'application dans laquelle débouche un conduit de sortie formant un orifice de distribution. De plus, la tête d'application comprend un logement pour une source lumineuse (led) émettant une lumière rouge ayant une longueur d'onde de l'ordre de 660 µm. Cet ensemble de distribution comprend un corps de réception dans lequel est intégrée une pompe. De plus, ce corps forme un logement pour accueillir le module générant la lumière rouge. Dans sa partie basse, l'ensemble de ce document comprend un étui amovible dans lequel est reçu un réservoir de produit fluide qui est monté de manière amovible à la pompe intégrée au corps. La seule partie amovible et remplaçable de cet ensemble est le réservoir de produit fluide reçu dans son étui. Il n'est pas possible d'extraire la pompe du corps, étant donné que la pompe est directement surmoulée à l'intérieur du corps. D'autre part, la surface d'application est uniquement et entièrement formée par la tête qui forme le conduit de sortie et l'orifice de distribution, ainsi que le logement de réception pour la source lumineuse.

On connaît également le document US2011/0190672 décrivant un applicateur vibrant comprenant un boîtier renfermant un réservoir pourvu d'un embout de distribution. Un collier disposé sur le boîtier renferme un élément vibrant qui entoure l'embout de distribution. Une tête de stockage thermique est couplée à l'élément vibrant pour la faire vibrer : la tête est mobile par rapport au boîtier. Cette tête définit une surface d'application dans laquelle débouche l'embout de distribution. Le collier comprend un poussoir latéral pour activer l'élément vibrant. La distribution du produit fluide s'effectue en écrasant le réservoir : une variante avec une pompe est évoquée sans rentrer dans le détail. Ce distributeur n'a pour vocation a été démonté et constitue ainsi un ensemble unitaire.

La présente invention a pour but d'améliorer ces ensembles de distribution et d'application de produit fluide de l'art antérieur en les rendant à la fois plus modulables, plus propres, moins chers et plus facilement adaptables aux diverses technologies de génération d'ondes.

Pour ce faire, la présente invention propose un ensemble de distribution et d'application de produit fluide comprenant un corps de réception creux, une surface d'application de produit fluide montée à une extrémité supérieure du corps de réception et destinée à venir en contact avec une cible, telle que la peau, un distributeur de produit fluide comprenant un réservoir, une pompe et un embout de distribution définissant une surface de sortie dans laquelle débouche un orifice de distribution de produit fluide caractérisé en ce que le distributeur est reçu de manière amovible dans le corps de réception avec son embout de distribution reçu de manière amovible dans un logement, sa surface de sortie formant une partie de la surface d'application, de sorte que l'orifice de distribution débouche directement dans la surface d'application, le distributeur étant extractible hors du corps de réception à travers une extrémité inférieure ouverte du corps de réception qui est opposée à l'extrémité supérieure.

Ainsi, le produit fluide chemine uniquement à l'intérieur du distributeur jusqu'au niveau de la surface d'application dont la surface de sortie forme également une partie de la surface d'application. Le produit fluide est de ce fait en contact qu'avec le distributeur et la surface d'application, à l'exclusion de tout autre élément de surface. Ceci n'est pas le cas dans l'ensemble de distribution du document WO2013/121145, dans lequel le produit fluide à la sortie de la pompe traverse une section du corps de réception et le conduit de sortie formé par la tête d'application. Avec la conception selon l'invention, l'ensemble de distribution et d'application est bien plus propre et plus facilement nettoyable. Dans la présente invention, c'est l'ensemble du distributeur, comprenant le réservoir, la pompe et l'embout de distribution, qui est reçu de manière amovible dans le corps de réception et dans le logement. Ainsi, on peut aisément retirer un distributeur du corps et le remplacer par un autre sans qu'il ne reste la moindre trace du produit fluide à l'intérieur de l'ensemble de distribution. Ceci est en effet possible, étant donné que le produit fluide ne chemine qu'à l'intérieur du distributeur jusqu'à la surface d'application, comme déjà mentionné précédemment. Par conséquent, avec le même ensemble de distribution et d'application de la présente invention, on peut distributeur plusieurs types de produit fluide en remplaçant simplement le distributeur de produit fluide, et cela sans risque de restes de produit fluide ou de mélanges de produit fluide. Il suffit à l'utilisateur de nettoyer correctement la surface d'application avant de retirer le distributeur de produit fluide pour y insérer un autre.

Selon un autre aspect avantageux de l'invention, l'embout de distribution et le logement présentent une section transversale correspondante imposant un engagement mutuel avec une orientation angulaire unique. Avantageusement, le distributeur définit un axe longitudinal qui passe par le réservoir, la pompe et l'embout de distribution, la pompe comprenant un actionneur latéral qui est mobile transversalement à l'axe longitudinal. Ainsi, l'ensemble peut être saisi et manipulé à la manière d'un stylo en appuyant sur l'actionneur latéral à l'aide de l'index. De préférence, l'embout de distribution est orienté et bloqué en rotation par rapport à l'actionneur latéral, le corps de réception comprenant un poussoir latéral qui est disposé en face de l'actionneur latéral pour pourvoir déplacer l'actionneur latéral en appuyant latéralement sur le poussoir latéral, l'orientation angulaire du distributeur dans le corps de réception étant imposée par l'engagement de l'embout de distribution dans le logement.

Selon un autre aspect très avantageux de l'invention, la surface d'application est partiellement formée par une entité d'application comprenant une tête d'application formant le logement, et un module de génération d'ondes qui émet des ondes et les transmet à la surface d'application, le module étant en partie reçu dans le corps de réception et formant avantageusement une partie de la surface d'application. Les ondes émises par le module de génération d'ondes peuvent être de toute nature, comme par exemple des rayonnements dans la lumière du visible, des rayonnements infrarouges ou ultraviolets, de manière générale toute sorte d'ondes électromagnétiques, ou encore des ondes vibratoires comme par exemple des ultrasons, ou des ondes mécaniques (massage), etc. Le module de génération d'ondes peut être associé à la tête d'application par n'importe quelle technique appropriée, comme par exemple par encliquetage, collage, emmanchage en force, surmoulage, collage, etc.

Avantageusement, l'entité d'application est reçue de manière amovible sur et dans le corps de réception, le module étant avantageusement encliqueté de manière amovible dans le corps de réception. Avec une telle conception, on comprend aisément que l'entité d'application est totalement indépendante du distributeur de produit fluide, hormis l'engagement de l'embout de distribution de manière amovible à l'intérieur du logement formé par la tête d'application. Il est donc possible de remplacer l'entité d'application en fonction du distributeur de produit fluide. On peut ainsi imaginer de prévoir plusieurs types différents de distributeurs de produit fluide auxquels sont associés respectivement différents types correspondants d'entités d'application comprenant des modules de génération d'ondes correspondants.

Selon une caractéristique très pratique de l'invention, la surface de sortie est disposée en affleurement avec la surface d'application pour la compléter de manière lisse, régulière et continue. Avantageusement, la surface de sortie présente un contour extérieur dont la forme correspond à celle du logement au moins au niveau de la surface d'application, de sorte que l'embout de distribution obture le logement de manière étanche, au moins au niveau de la surface d'application. On garantit ainsi que la surface de sortie est parfaitement intégrée à la surface d'application sans possibilité d'infiltration de produit fluide à l'intérieur du logement. De ce fait, en nettoyant très simplement la surface d'application, par exemple par frottement ou essuyage, on peut retirer le distributeur de produit fluide de l'ensemble sans qu'il ne reste aucune trace de produit fluide dans le corps de réception, le logement ou sur la surface d'application.

Selon un autre aspect intéressant de la présente invention, l'ensemble peut comprendre un fond amovible qui coopère avec le corps de réception pour obturer l'extrémité inférieure, ce fond poussant, avantageusement de manière élastique, le distributeur vers la tête d'application de manière à appuyer l'embout de distribution dans le logement. De préférence, le réservoir pénètre dans le fond amovible de sorte que le réservoir fait saillie hors du corps de réception après retrait du fond pour pouvoir saisir le réservoir et extraire le distributeur hors du corps de réception. On garantit non seulement l'étanchéité de l'embout de distribution dans le logement, mais également un maintien stable du distributeur à l'intérieur du corps de réception, tout en permettant son extraction aisée.

Selon une autre approche avantageuse de l'invention, on peut noter que l'ensemble comprend trois entités distinctes qui sont assemblées de manière amovible, à savoir une entité de réception formée par le corps de réception, avantageusement pourvu d'un poussoir et d'un fond amovible, une entité d'application formant la surface d'application et comprenant une tête d'application et un module de génération d'ondes, et une entité de distribution constituée par le distributeur de produit fluide. On perçoit alors directement que l'ensemble résulte de l'assemblage de trois entités distinctes qui procurent à la fois une modularité, une facilité de nettoyage, une facilité de montage, et de ce fait un coût réduit pour la fabrication de l'ensemble. Avantageusement, la pompe est disposée entre le poussoir et le module. Grâce à cette conception compacte, l'ensemble peut se présenter sous la forme d'un stylo avec une partie bombée où sont disposés la pompe, le poussoir et le module.

L'esprit de l'invention réside dans le fait que le produit fluide ne sort du distributeur qu'au niveau de la surface d'application, et ceci est d'autant plus avantageux que le distributeur est monté de manière amovible et que la surface d'application est au moins partiellement formée par une entité d'application intégrant également un module de génération d'ondes. La décomposition de l'ensemble en trois entités distinctes est également une caractéristique intéressante, car elle permet de dissocier le distributeur du module de génération d'ondes, qui peuvent ainsi être conçus et fabriqués par des sociétés différentes.

L'invention sera maintenant décrite plus en détail en référence aux dessins joints, donnant à titre d'exemple non limitatif, un mode de réalisation de l'invention.

Sur les figures :
La figure 1 est une vue en section transversale verticale à travers un ensemble de distribution et d'application de produit fluide de l'invention, sensiblement à l'échelle 1,
La figure 2 est une vue fortement agrandie de la partie supérieure de la figure 1,
La figure 3 est une vue en section transversale horizontale le long de la ligne de section A-A de la figure 2,
La figure 4 est une vue en perspective explosée de l'ensemble de distribution et d'application des figures précédentes,
La figure 5 est une vue similaire à celle de la figure 1, en l'absence de distributeur de produit fluide,
La figure 6 est une vue en section transversale verticale à travers un distributeur de produit fluide selon l'invention,
La figure 7 est une vue de dessus du distributeur de la figure 6, et
La figure 8 est une vue en perspective explosée du distributeur des figures 6 et 7.

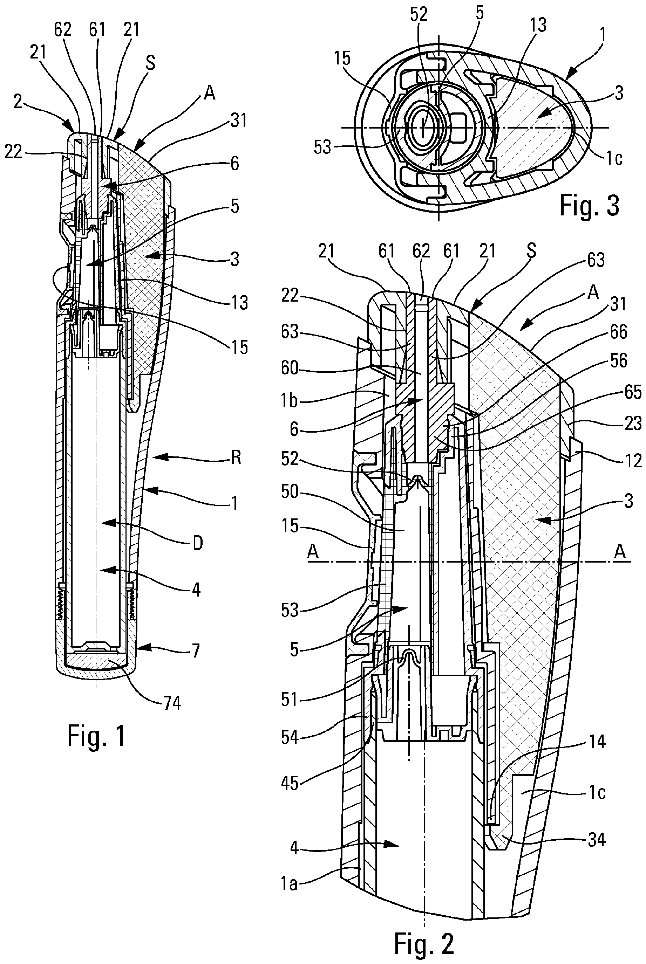

Comme on peut le voir à partir des différentes figures qui illustrent la présente invention, l'ensemble de distribution et d'application de produit fluide de l'invention présente une forme allongée ou élancée qui peut s'apparenter à celle d'un stylo ou d'un feutre. On peut également noter que sa section transversale n'est pas constante, puisqu'elle varie de bas en haut de manière significative. En l'occurrence, à proximité de son extrémité inférieure, l'ensemble de distribution et d'application présente une section transversale plutôt ronde ou circulaire, alors qu'au niveau de la ligne de coupe A-A, qui est plutôt située à proximité de l'extrémité supérieure, l'ensemble de distribution et d'application présente une section transversale de forme ovoïdale (Figure 3). La face supérieure de l'ensemble forme une surface d'application S qui présente une inclinaison ou pente vers un côté.

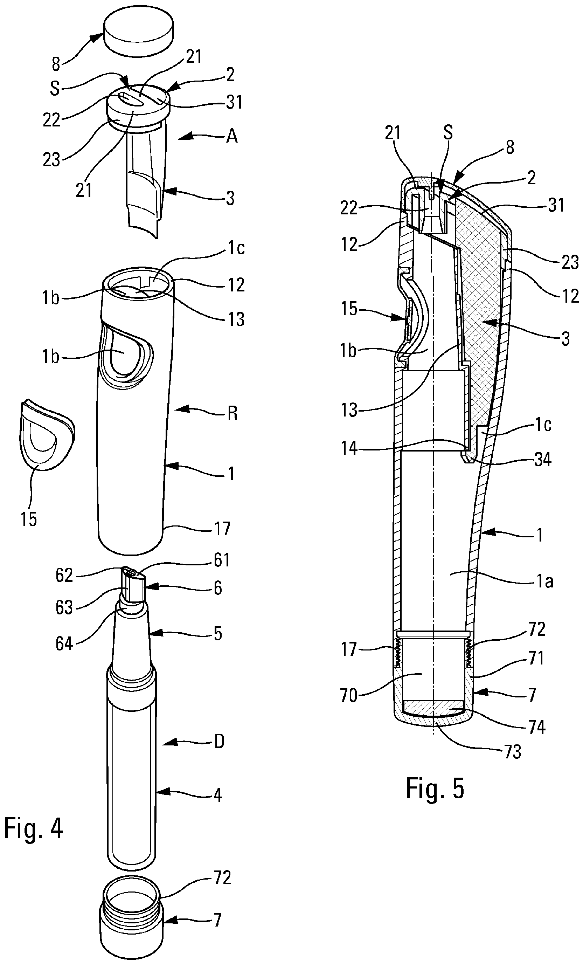

En se référant à la figure 4, on peut voir les différents éléments constitutifs de l'ensemble de distribution et d'application de l'invention. On peut tout d'abord remarquer qu'il comprend trois entités principales distinctes, à savoir une entité de distribution D, une entité de réception R et une entité d'application A. L'entité de distribution D, qui est un distributeur de produit fluide, est reçue, avantageusement de manière amovible, à l'intérieur de l'entité de réception R, qui comprend un corps monobloc de réception 1 dont l'intérieur est creux. L'entité d'application A est montée sur et dans le corps 1, avantageusement de manière amovible. Ainsi de manière préférentielle, les deux entités D et A sont reçues de manière amovible sur et dans le corps 1 à partir de ces deux extrémités opposées 17 et 12, respectivement. Il s'agit là de l'architecture globale de l'ensemble de distribution et d'application selon l'invention.

Plus en détail, le corps 1 de l'entité de réception R est ouvert à ses deux extrémités haute 12 et basse 17 pour pouvoir accueillir les entités A et D. L'extrémité basse 17 est avantageusement filetée intérieurement pour recevoir par vissage un fond amovible 7. Ce dernier présente la forme d'un petit godet avec une paroi inférieure 73 et une paroi latérale cylindrique 71 dont la partie supérieure forme un filetage 72 dont le pas correspond à celui de l'extrémité basse 17 du corps 1. On peut remarquer que le fond amovible 7 est pourvu au-dessus de sa paroi inférieure 73 d'une pièce de matière élastique 74, qui peut être une mousse ou un élastomère. Le fond amovible 7 forme intérieurement un espace 70 qui communique vers le haut avec l'intérieur du corps 1 qui définit lui-même un espace de réception 1a. Au-delà de cet espace de réception 1a, l'intérieur du corps 1 est divisé en deux compartiments 1b et 1c par une cloison de séparation 13. Le compartiment 1b s'étend axialement dans le prolongement de l'espace 1a, alors que le compartiment 1c s'étend latéralement, au niveau où le corps 1 définit sa forme ovoïdale. L'extrémité inférieure de la cloison 13 forme une arête d'encliquetage 14 comme on le verra ci après. Le corps 1, au niveau du compartiment 1b est pourvu d'un poussoir latéral 15 qui est déplaçable transversalement à l'axe longitudinal du corps de réception 1. Le déplacement du poussoir 15 peut se faire par translation pure ou par déformation élastique. On peut par exemple prévoir de surmouler le poussoir 15 sur le corps de réception 1 avec une matière élastomérique. En variante, on peut également prévoir un poussoir 15 qui se déplace totalement indépendamment du corps 1. On peut aussi prévoir une simple ouverture à la place du poussoir 15. On peut également remarquer que la cloison de séparation 13 s'étend jusqu'à proximité de l'extrémité haute 12. Le corps de réception 1 peut très simplement être réalisé par injection moulage de matière plastique, ou même de métal.

L'entité d'application A résulte ici de l'association d'une tête d'application 2 et d'un module de génération d'ondes 3. La tête d'application 2, comme on peut le voir sur les figures 1 et 2, comprend un logement axial 22 formé par une tubulure cylindrique dont la section transversale présente une forme géométrique complexe, par exemple celle d'un croissant. Ce logement 22 se raccorde vers le haut à une plage de surface d'application 21 qui est ici formée avec deux ouvertures, à savoir une première ouverture correspondant à l'embouchure du logement 22 et une seconde ouverture pour le module 3. Plus précisément, le module 3 comprend une section de surface d'application 31 qui obture l'ouverture correspondante de la tête 2 de manière à compléter de manière continue et lisse la plage de surface d'application 21 de la tête 2. En d'autres termes, le module 3 s'adapte dans l'ouverture de la tête d'application de sorte que la section de surface d'application 31 du module 3 complète la plage de surface d'application de la tête 2 sans créer de discontinuité, de saillant ou de creux. Par conséquent, l'assemblage du module 3 et de la tête 2 permet de créer une surface d'application S dont la seule ouverture, à ce stade, est formée par l'embouchure du logement 22. On peut remarquer sur les figures 1 et 2 que la section de surface d'application 31 s'étend au niveau de la partie la plus inclinée de la surface d'application S. La tête d'application 2 comprend également une jupe périphérique 23 qui vient s'adapter dans l'extrémité haute 12 du corps creux 1. D'autre part, le module de génération d'ondes 3 s'étend à l'intérieur de l'espace de réception 1c, et présente avantageusement un profil d'encliquetage 34 apte à coopérer avec le bord inférieur 14 de la cloison de séparation 13. De cette manière, l'entité d'application A peut être montée de manière parfaitement stable sur et dans el corps creux 1.

Selon l'invention, le module de génération d'ondes 3 permet de générer tout type d'ondes ou de rayonnement électromagnétique, vibratoire, etc., comme par exemple de la lumière visible, des infrarouges, des ultraviolets, voire des micro-ondes, etc. ou encore des ultrasons ou des vibrations mécaniques. Le module 3 peut également générer de la chaleur ou du froid (ondes thermiques) pour conférer un effet chaud ou froid au contact avec la peau.

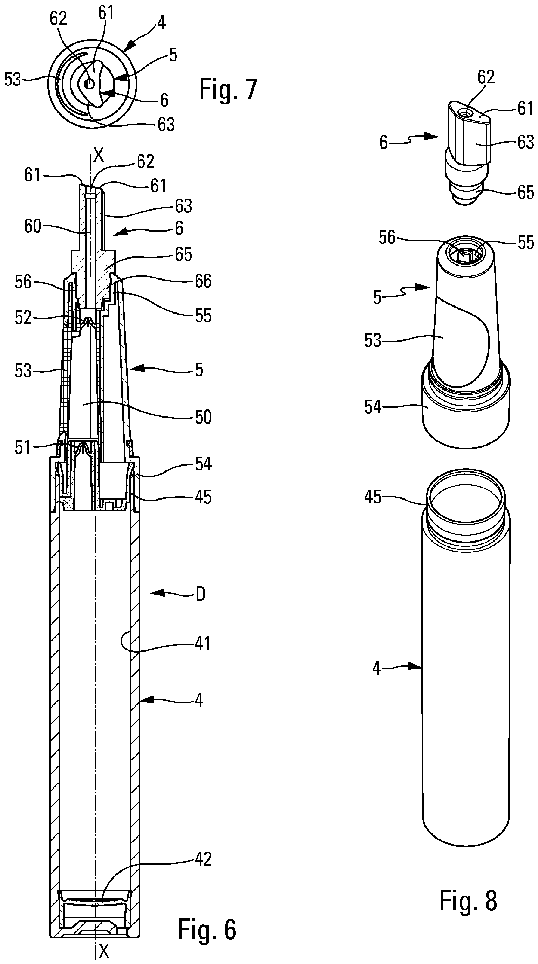

L'entité de distribution ou distributeur de produit fluide D comprend un réservoir de produit fluide 4, une pompe 5 et un embout de distribution 6, comme on peut le voir plus clairement sur les figures 6 à 8.

Le réservoir 4 peut par exemple se présenter sous la forme d'un fût de coulissement 41 dans lequel est reçu un piston suiveur 42 qui est adapté à coulisser dans le fût 41 à mesure que du produit fluide est extrait du réservoir. Le sommet du fût 41 forme un col 45. A la place de ce réservoir particulier, on peut également imaginer un réservoir plus simple sans variation de volume utile, ou encore un réservoir avec une poche souple.

La pompe 5 comprend une bague de fixation 54 permettant son montage sur le col 45 du réservoir 4. La pompe 5 comprend une chambre de pompe 50 pourvue à son extrémité inférieure d'un clapet d'entrée 51, par exemple sous la forme d'un obturateur à fente. A son extrémité supérieure, la chambre de pompe 50 comprend un clapet de sortie 52, qui peut par exemple être également réalisé sous la forme d'un obturateur à fente. En outre, la chambre de pompe 50 comprend un actionneur latéral 53 qui permet de réduire le volume utile de la chambre de pompe 50 et ainsi de refouler du produit fluide à travers le clapet de sortie 52. L'actionneur latéral 53 est déplaçable perpendiculairement à l'axe longitudinal X du distributeur D. Le déplacement peut se faire translativement ou encore par déformation élastique. Dans le mode de réalisation utilisé pour illustrer la présente invention, l'actionneur 53 se présente sous la forme d'une paroi souple de la chambre de pompe 50 qui a été réalisée par exemple par un procédé de bi-injection ou de surmoulage. La pompe 5 peut ainsi être qualifiée de pompe à membrane souple, en ce sens que l'on agit directement sur une paroi mobile de la chambre pour mettre le produit fluide sous pression. A son extrémité supérieure, la pompe 5 forme un puits de montage 56 pour l'embout de distribution 6. Ce puits de montage 56 est avantageusement pourvu de moyens de détrompage 55, par exemple sous la forme d'un profil saillant ou d'un évidement, permettant d'imposer l'orientation angulaire de l'embout 6 dans le puits 56.

L'embout de distribution 6 comprend ainsi un talon de montage 65 qui est engagé, et avantageusement encliqueté, à l'intérieur du puits de montage 56. Le talon de montage 65 comprend un profil de détrompage qui s'adapte parfaitement dans les moyens de détrompage 55 du puits 56, afin d'imposer l'orientation angulaire de l'embout de distribution 6 sur la pompe 5. De cette manière, l'embout est toujours orienté de la même manière par rapport à l'actionneur latéral 53, qui ne s'étend que sur un côté de la pompe 5. Au-dessus de ce talon de montage 65, l'embout de distribution 6 forme un appendice d'insertion 63 dont la section transversale présente une forme correspondante à celle du logement 22 formé par la tête d'application 2. Cette forme est plus clairement visible sur la figure 7 : elle s'apparente à une forme d'un croissant. La paroi latérale de l'appendice d'insertion 63 peut être cylindrique, bien que non circulaire sur toute sa hauteur. En variante, on peut prévoir un ou plusieurs cordon(s) d'étanchéité saillant(s) permettant de réaliser une étanchéité à l'intérieur du logement 22. A son extrémité supérieure, l'appendice 63 forme une surface de sortie sensiblement plane 61 qui est percée d'un orifice de distribution 62, formant la sortie d'un conduit de sortie 60 qui traverse l'appendice 63 et le talon de montage 65, comme on peut clairement le voir sur les figures 2 et 6.

Une fois l'embout de distribution 6 monté sur la pompe 5, comme visible sur la figure 6, on peut voir que le clapet de sortie 52 communique directement avec le conduit de sortie 60. Ainsi, en enfonçant l'actionneur latéral 53, on réduit le volume utile de la chambre de pompe 50, et du produit fluide sous pression est refoulé à travers le clapet de sortie 52, d'où il peut s'écouler à travers le conduit de sortie 60 jusqu'à l'orifice de distribution 62 situé au niveau de la surface de sortie 61. Dès que l'on relâche la pression sur l'actionneur latéral 53, le clapet de sortie 52 se ferme et le clapet d'entrée 51 s'ouvre sous l'effet de la dépression créée dans la chambre de pompe 50, permettant ainsi d'aspirer du produit fluide en provenance du réservoir 4, dont le piston suiveur 42 se déplace alors vers la pompe 5.

Comme on peut le comprendre à partir de la figure 4, l'entité de distribution du distributeur D est insérée à l'intérieur du corps creux 1 à travers son extrémité basse 17, après retrait du fond amovible 7. Le distributeur D est ainsi inséré axialement à travers l'espace 1a, puis à travers l'espace 1b jusqu'à ce que l'embout de distribution 6 pénètre à l'intérieur du logement 22 de la tête d'application 2. Comme précédemment expliqué, il est nécessaire d'orienter angulairement le distributeur D afin que son appendice d'insertion 63 s'engage à l'intérieur du logement 22. L'orientation angulaire est de préférence unique. Il est alors possible d'engager à fond l'appendice 63 à l'intérieur du logement 22 jusqu'à ce que la surface de sortie 61 parvienne au niveau de la surface d'application S de manière à la compléter. Ceci est visible sur la figure 2. On peut voir que la surface de sortie 61 vient parfaitement en affleurement avec la plage de surface d'application 21 de la tête 2 de manière à la compléter. Au final, seul l'orifice de distribution 62 rompt la continuité de la surface d'application S. Pour garantir l'engagement à fond de l'appendice 63 dans le logement 22, on se sert du fond amovible 7 dont la matière souple 74 vient en contact du fond du réservoir 4 pour le pousser vers le haut, et réaliser une étanchéité au niveau du logement 22. Il faut à cet égard également remarquer que l'extrémité inférieure du réservoir 4 fait saillie hors du corps creux 1 lorsque le fond amovible 7 est retiré, de manière à pouvoir aisément saisir le distributeur par son réservoir 4 pour l'extraire du corps creux 1. De ce fait, le distributeur D est reçu de manière amovible à l'intérieur du corps creux 1 et de la tête 2. On peut également remarquer que l'orientation angulaire imposée de l'appendice 63 à l'intérieur du logement 22 permet de disposer l'actionneur latéral 53 en face du poussoir 15 du corps creux 1.

Sur la figure 3, on peut voir la disposition des différents éléments constitutifs de l'ensemble de distribution et d'application de l'invention au niveau où il présente sa forme ovoïdale. On peut par exemple voir que le module de génération 3 est reçu à l'intérieur du compartiment 1c qui est délimité par la cloison de séparation 13 qui entoure partiellement la pompe 5 dont l'actionneur latéral 53 est recouvert par le poussoir latéral 15. On peut ainsi dire que la pompe 5 est disposée entre le module 3 et le poussoir 15 à l'intérieur du corps creux 1.

Avec une telle conception, l'entité d'application A est reçue de manière amovible sur et dans l'entité de réception R. D'autre part, l'entité de distribution du distributeur D est également reçue de manière amovible à l'intérieur de l'entité de réception R et à l'intérieur du logement 22 de l'entité d'application A. De cette manière, on peut remplacer à volonté le distributeur D et l'entité d'application A en fonction des besoins. On peut par exemple imaginer qu'un distributeur particulier distribuant un produit fluide particulier est associé à une entité d'application particulière. Il suffit alors de monter les deux entités A et D dans l'entité de réception R pour constituer l'ensemble de distribution et d'application de l'invention. Au cas où il est nécessaire de remplacer les entités A et D, cela est aisément possible en les retirant chacune de l'entité de réception R.

Il faut aussi remarquer que le produit fluide distribué par le distributeur D ne sort du distributeur qu'au niveau de la surface d'application S, de sorte qu'il ne peut rester de produit fluide à l'intérieur de l'entité de réception R, une fois le distributeur retiré. De plus, du fait que la surface d'application S est parfaitement continue et lisse, elle est aisément nettoyable par frottement ou essuyage. Ainsi, lorsqu'un utilisateur veut changer de distributeur, il lui suffit au préalable de nettoyer la surface d'application S, puis de retirer le distributeur et de le remplacer par un autre. Aucune souillure ou dépôt de produit fluide ne peut être observé.

Dans le mode de réalisation utilisé pour illustrer la présente invention, le module de génération d'ondes 3 forme un secteur de surface d'application 31. Il ne s'agit là que d'une forme de réalisation particulière non limitative, car il est tout à fait envisageable de réaliser le module de génération d'ondes 3 sans qu'il ne forme une partie de la surface d'application S. Le module 3 peut par exemple être associé à la tête d'application 2 juste en dessous de la surface d'application S, qui va alors servir de moyens de diffusion pour les ondes.

L'indépendance totale entre le distributeur D et l'entité d'application A, hormis lors de l'assemblage dans le logement 22, permet de dissocier totalement ces deux entités, de sorte qu'elles peuvent être produites par des sociétés tout à fait différentes, à savoir une société spécialisée dans la conception de distributeurs et une société spécialisée dans la conception de modules électroniques générateurs d'ondes.

Grâce à l'invention, on obtient un ensemble de distribution et d'application de produit fluide comprenant trois entités distinctes et dont la propreté et le nettoyage sont aisément garantis.

## Revendications

1. Ensemble de distribution et d'application de produit fluide comprenant :
- un corps de réception (1) creux,
- une surface d'application de produit fluide (S) montée à une extrémité supérieure (12) du corps de réception (1) et destinée à venir en contact avec une cible, telle que la peau,
- un distributeur de produit fluide (D) comprenant un réservoir (4), une pompe (5) et un embout de distribution (6) définissant une surface de sortie (61) dans laquelle débouche un orifice de distribution de produit fluide (62),
**caractérisé en ce que** le distributeur (D) est reçu de manière amovible dans le corps de réception (1) avec son embout de distribution (6) reçu de manière amovible dans un logement (22), sa surface de sortie (61) formant une partie de la surface d'application (S), de sorte que l'orifice de distribution (62) débouche directement dans la surface d'application (S), le distributeur (D) étant extractible hors du corps de réception (1) à travers une extrémité inférieure (17) ouverte du corps de réception (1) qui est opposée à l'extrémité supérieure (12).

2. Ensemble selon la revendication 1, dans lequel l'embout de distribution (6) et le logement (22) présentent une section transversale correspondante imposant un engagement mutuel avec une orientation angulaire unique.

3. Ensemble selon l'une quelconque des revendications précédentes, dans lequel le distributeur (D) définit un axe longitudinal X qui passe par le réservoir (4), la pompe (5) et l'embout de distribution (6), la pompe (5) comprenant un actionneur latéral (53) qui est mobile transversalement à l'axe longitudinal X.

4. Ensemble selon la revendication 3, dans lequel l'embout de distribution (6) est orienté et bloqué en rotation par rapport à l'actionneur latéral (53), le corps de réception (1) comprenant un poussoir latéral (15) qui est disposé en face de l'actionneur latéral (53) pour pourvoir déplacer l'actionneur latéral (53) en appuyant latéralement sur le poussoir latéral (15), l'orientation angulaire du distributeur (D) dans le corps de réception (1) étant imposée par l'engagement de l'embout de distribution (6) dans le logement (22).

5. Ensemble selon l'une quelconque des revendications précédentes, dans lequel la surface de sortie (61) présente un contour extérieur dont la forme correspond à celle du logement (22) au moins au niveau de la surface d'application (S), de sorte que l'embout de distribution (6) obture le logement (22) de manière étanche, au moins au niveau de la surface d'application (S).

6. Ensemble selon l'une quelconque des revendications précédentes, dans lequel la surface d'application (S) est partiellement formée par une entité d'application (A) comprenant une tête d'application (2) formant le logement (22), et un module de génération d'ondes (3) qui émet des ondes et les transmet à la surface d'application (S), le module (3) étant en partie reçu dans le corps de réception (1) et formant avantageusement une partie de la surface d'application (S).

7. Ensemble selon la revendication 6, dans lequel l'entité d'application (A) est reçue de manière amovible sur et dans le corps de réception (1), le module (3) étant avantageusement encliqueté de manière amovible dans le corps de réception (1).

8. Ensemble selon l'une quelconque des revendications précédentes, dans lequel la surface de sortie (61) est disposée en affleurement avec la surface d'application (S) pour la compléter de manière lisse, régulière et continue.

9. Ensemble selon l'une quelconque des revendications précédentes, comprenant un fond amovible (7) qui coopère avec le corps de réception (1) pour obturer l'extrémité inférieure (17), ce fond amovible (7) poussant, avantageusement de manière élastique, le distributeur (D) vers la tête d'application (2) de manière à appuyer l'embout de distribution (6) dans le logement (22).

10. Ensemble selon la revendication 9, dans lequel le réservoir (4) pénètre dans le fond amovible (7) de sorte que le réservoir (4) fait saillie hors du corps de réception (1) après retrait du fond amovible (7) pour pouvoir saisir le réservoir (4) et extraire le distributeur (D) hors du corps de réception (1).

11. Ensemble selon l'une quelconque des revendications précédentes, comprenant trois entités distinctes qui sont assemblées de manière amovible, à savoir :
- une entité de réception (R) formée par le corps de réception (1), avantageusement pourvu d'un poussoir (15) et d'un fond amovible (7),
- une entité d'application (A) formant la surface d'application (S) et comprenant une tête d'application (2) et un module de génération d'ondes (3), et
- une entité de distribution (D) constituée par le distributeur de produit fluide.

12. Ensemble selon la revendication 11, dans lequel la pompe (5) est disposée entre le poussoir (15) et le module de génération d'ondes (3).

## Patentansprüche

1. Anordnung zur Ausgabe und Applikation eines fluiden Produkts, aufweisend:
- einen hohlen Aufnahmekörper (1),
- eine Auftragsfläche für ein fluides Produkt (S), die an einem oberen Ende (12) des Aufnahmekörpers (1) montiert und dafür gedacht ist, mit einem Ziel, wie Haut, in Kontakt zu kommen,
- einen Spender für ein fluides Produkt (D), aufweisend einen Behälter (4), eine Pumpe (5) und einen Ausgabeansatz (6), der eine Ausgabefläche (61) definiert, in der eine Ausgabeöffnung für ein fluides Produkt (62) mündet,
**dadurch gekennzeichnet, dass** der Spender (D) abnehmbar in dem Aufnahmekörper (1) aufgenommen ist, wobei sein Ausgabeansatz (6) abnehmbar in einer Aufnahme (22) aufgenommen ist, wobei seine Ausgabefläche (61) einen Teil der Auftragsfläche (S) derart bildet, dass die Ausgabeöffnung (62) direkt in der Auftragsfläche (S) mündet, wobei der Spender (D) über ein unteres offenes Ende (17) des Aufnahmekörpers (1), das dem oberen Ende (12) entgegengesetzt ist, aus dem Aufnahmekörper (1) herausziehbar ist.

2. Anordnung nach Anspruch 1, wobei der Ausgabeansatz (6) und die Aufnahme (22) einen entsprechenden Querschnitt haben, der einen gegenseitigen Eingriff mit einer eindeutigen Winkelausrichtung bewirkt.

3. Anordnung nach einem der vorhergehenden Ansprüche, wobei der Spender (D) eine Längsachse X definiert, die durch den Behälter (4), die Pumpe (5) und den Ausgabeansatz (6) hindurch geht, wobei die Pumpe (5) eine seitliche Betätigungseinrichtung (53) aufweist, die quer zur Längsachse X beweglich ist.

4. Anordnung nach Anspruch 3, wobei der Ausgabeansatz (6) in Bezug auf die seitliche Betätigungseinrichtung (53) ausgerichtet und drehfest ist, wobei der Aufnahmekörper (1) eine seitliche Druckvorrichtung (15) aufweist, die gegenüber der seitlichen Betätigungseinrichtung (53) angeordnet ist, um die seitliche Betätigungseinrichtung (53) verschieben zu können, indem seitlich auf die seitliche Druckvorrichtung (15) gedrückt wird, wobei die Winkelausrichtung des Spenders (D) in dem Aufnahmekörper (1) durch den Eingriff des Ausgabeansatzes (6) in der Aufnahme (22) bewirkt wird.

5. Anordnung nach einem der vorhergehenden Ansprüche, wobei die Ausgabefläche (61) eine Außenkontur aufweist, deren Form derjenigen der Aufnahme (22) zumindest an der Auftragsfläche (S) derart entspricht, dass der Ausgabeansatz (6) die Aufnahme (22) zumindest an der Auftragsfläche (S) dichtend verschließt.

6. Anordnung nach einem der vorhergehenden Ansprüche, wobei die Auftragsfläche (S) teilweise durch eine Auftragseinheit (A) gebildet ist, die einen Auftragskopf (2), der die Aufnahme (22) bildet, und ein Wellenerzeugungsmodul (3) aufweist, das Wellen ausgibt und sie auf die Auftragsfläche (S) überträgt, wobei das Modul (3) zum Teil in dem Aufnahmekörper (1) aufgenommen ist und vorteilhafterweise einen Teil der Auftragsfläche (S) bildet.

7. Anordnung nach Anspruch 6, wobei die Auftragseinheit (A) abnehmbar auf und in dem Aufnahmekörper (1) aufgenommen ist, wobei das Modul (3) vorteilhafterweise abnehmbar in dem Aufnahmekörper (1) eingerastet ist.

8. Anordnung nach einem der vorhergehenden Ansprüche, wobei die Ausgabefläche (61) mit der Auftragsfläche (S) bündig angeordnet ist, um sie glatt, gleichmäßig und durchgängig zu vervollständigen.

9. Anordnung nach einem der vorhergehenden Ansprüche, aufweisend einen abnehmbaren Boden (7), der mit dem Aufnahmekörper (1) zusammenwirkt, um das untere Ende (17) zu verschließen, wobei dieser abnehmbare Boden (7) den Spender (D) vorteilhafterweise elastisch derart in Richtung zum Auftragskopf (2) schiebt, dass er den Ausgabeansatz (6) in die Aufnahme (22) drückt.

10. Anordnung nach Anspruch 9, wobei der Behälter (4) derart in den abnehmbaren Boden (7) eindringt, dass der Behälter (4) nach dem Einziehen des abnehmbaren Bodens von dem Aufnahmekörper (1) vorsteht, um den Behälter (4) ergreifen und den Spender (D) aus dem Aufnahmekörper (1) herausziehen zu können.

11. Anordnung nach einem der vorhergehenden Ansprüche, aufweisend drei unterschiedliche Einheiten, die abnehmbar zusammengebaut sind, nämlich:
- eine Aufnahmeeinheit (R), die von dem Aufnahmekörper (1) gebildet und vorteilhafterweise mit einer Druckvorrichtung (15) und einem abnehmbaren Boden (7) versehen ist,
- eine Auftragseinheit (A), die die Auftragsfläche (S) bildet und einen Auftragskopf (2) und ein Wellenerzeugungsmodul (3) aufweist, und
- eine Ausgabeeinheit (D), die von dem Spender für ein fluides Produkt gebildet ist.

12. Anordnung nach Anspruch 11, wobei die Pumpe (5) zwischen der Druckvorrichtung (15) und dem Wellenerzeugungsmodul (3) angeordnet ist.

## Claims

1. A fluid dispenser and applicator assembly comprising:
• a hollow reception body (1);
• a fluid applicator surface (S) that is mounted at a top end (12) of the reception body (1) and that is for coming into contact with a target, such as the skin;
• a fluid dispenser (D) comprising a reservoir (4), a pump (5), and a dispenser endpiece (6) that defines an outlet surface (61) in which a fluid dispenser orifice (62) opens out;
the assembly being **characterized in that** the dispenser (D) is received in removable manner in the reception body (1) with its dispenser endpiece (6) being received in removable manner in a housing (22), its outlet surface (61) forming a portion of the applicator surface (S), such that the dispenser orifice (62) opens out directly in the applicator surface (S), the dispenser (D) being removable from the reception body (1) through an open bottom end (17) of the reception body (1), which bottom end is remote from the top end (12).

2. An assembly according to claim 1, wherein the dispenser endpiece (6) and the housing (22) present a corresponding cross-section imposing mutual engagement with a single angular orientation.

3. An assembly according to any preceding claim, wherein the dispenser (D) defines a longitudinal axis X that passes via the reservoir (4), the pump (5), and the dispenser endpiece (6), the pump (5) including a lateral actuator (53) that is movable transversally relative to the longitudinal axis X.

4. An assembly according to claim 3, wherein the dispenser endpiece (6) has a particular orientation and is prevented from turning relative to the lateral actuator (53), the reception body (1) including a lateral pusher (15) that is arranged facing the lateral actuator (53) so as to make it possible to move the lateral actuator (53) by pressing laterally on the lateral pusher (15), the angular orientation of the dispenser (D) in the reception body (1) being imposed by the engagement of the dispenser endpiece (6) in the housing (22).

5. An assembly according to any preceding claim, wherein the outlet surface (61) presents an outline having a shape that corresponds to the shape of the housing (22) at least at the applicator surface (S), such that the dispenser endpiece (6) closes the housing (22) in sealed manner, at least at the applicator surface (S).

6. An assembly according to any preceding claim, wherein the applicator surface (S) is formed, in part, by an applicator unit (A) comprising an applicator head (2) that forms the housing (22), and a wave-generator module (3) that emits waves and transmits them to the applicator surface (S), the module (3) being received, in part, in the reception body (1) and advantageously forming a portion of the applicator surface (S).

7. An assembly according to claim 6, wherein the applicator unit (A) is received in removable manner on and in the reception body (1), the module (3) advantageously being snap-fastened in removable manner in the reception body (1).

8. An assembly according to any preceding claim, wherein the outlet surface (61) is arranged flush with the applicator surface (S) so as to complete it in smooth, regular, and continuous manner.

9. An assembly according to any preceding claim, including a removable bottom wall (7) that co-operates with the reception body (1) so as to close the bottom end (17), the removable bottom wall (7) acting, advantageously in resilient manner, to push the dispenser (D) towards the applicator head (2) so as to press the dispenser endpiece (6) into the housing (22).

10. An assembly according to claim 9, wherein the reservoir (4) penetrates into the removable bottom wall (7) so that the reservoir (4) projects out from the reception body (1) after the removable bottom wall (7) has been removed, enabling the reservoir (4) to be gripped and enabling the dispenser (D) to be removed from the reception body (1).

11. An assembly according to any preceding claim, comprising three distinct units that are assembled together in removable manner, namely:
• a reception unit (R) that is formed by the reception body (1) and that is advantageously provided with a pusher (15) and with a removable bottom wall (7);
• an applicator unit (A) that forms the applicator surface (S) and that comprises an applicator head (2) and a wave-generator module (3); and
• a dispenser unit (D) that is constituted by the fluid dispenser.

12. An assembly according to claim 11, wherein the pump (5) is arranged between the pusher (15) and the wave-generator module (3).
